19 Europäisches Patentamt

European Patent Office

Office européen des brevets

11 Publication number: **0 397 137 B1**

12 **EUROPEAN PATENT SPECIFICATION**

45 Date of publication of patent specification: **23.02.94** 51 Int. Cl.5: **C07C 50/34**, C07C 46/00

21 Application number: **90108725.4**

22 Date of filing: **09.05.90**

The file contains technical information submitted after the application was filed and not included in this specification

54 **Production process of quinizarin.**

30 Priority: **10.05.89 JP 118088/89**

43 Date of publication of application:
**14.11.90 Bulletin 90/46**

45 Publication of the grant of the patent:
**23.02.94 Bulletin 94/08**

84 Designated Contracting States:
**CH DE GB LI**

56 References cited:
**EP-A- 0 038 777**
**EP-B- 0 007 051**
**US-A- 2 445 538**

73 Proprietor: **SUMITOMO CHEMICAL COMPANY,
LIMITED**
**5-33, Kitahama 4-chome**
**Chuo-ku**
**Osaka(JP)**

72 Inventor: **Hattori, Makoto**
**1-3-5, Kitaochiai,**
**Suma-ku**
**Kobe-shi, Hyogo 654-01(JP)**
Inventor: **Yokoyama, Kaneo**
**3-1-48, Chiyogaoka**
**Nara-shi, Nara 631(JP)**

74 Representative: **VOSSIUS & PARTNER**
**Postfach 86 07 67**
**D-81634 München (DE)**

## Description

The present invention relates to an improvement in a process for producing quinizarin which is an important intermediate in dyestuff industry.

Quinizarin, 1,4-dihydroxyanthraquinone, is prepared by heating p-chlorophenol with phthalic anhydride in sulfuric acid in the presence of boric acid, but a large amount of waste acids is by-produced ( U S-A-2,445,538).

One of the approaches to reduce the amount of sulfuric acid to be used in the reaction above is to use fuming sulfuric acid which contains free $SO_3$ not less than 25% by weight or 10% by Weight (JP-B-83-8373 and JP-A-80-13289 which corresponds to EP-B-0 007 051). Although this approach is suitable to reduce the amount of the sulfuric acid used in the reaction to about 2 kg or less per kg of quinizarin, yield of quinizarin was as small as 75% or less and purity thereof was 87% at the highest as far as the inventors carried out the process described in the above-mentioned literature. Such a product is not suitable for the production of dyestuffs without further purification.

It is the object of the present invention to provide an improved process for producing quinizarin, wherein the amount of sulfuric acid to be used is small without lowering the yield or the purity of the product. This object could surprisingly be achieved by using a specific fuming sulfuric acid.

According to the present invention, a novel process for preparation of quinizarin is provided, wherein p-chlorophenol is allowed to react with phthalic anhydride in the fuming sulfuric acid given below in the presence of boric anhydride or boric acid, and the reaction product is hydrolyzed, the fuming sulfuric acid satisfying the formulas (1), (2) and (3), simultaneously,

$$6.20 - 0.090X \leqq Y \leqq 8.00 - 0.125X \quad (1)$$

$$X \leqq 24.0 \quad (2)$$

$$Y \leqq 5.25 \quad (3)$$

wherein X: content of free $SO_3$ (% by weight) in the fuming sulfuric acid, and
Y: molar ratio of the fuming sulfuric acid to the p-chlorophenol, and calculated according to the following equation.

$$Y = \frac{Z \left\{ \dfrac{X}{80.06} + \dfrac{100 - X}{98.08} \right\}}{\dfrac{100 \times W}{128.56}}$$

wherein
Z:      weight of fuming sulfuric acid used in the reaction,
W:      weight of p-chlorophenol used in the reaction,
and
X:      defined above.

When $B_2O_3$ is used, "content of free $SO_3$ (% by weight) in the fuming acid" is the actual content of free $SO_3$ (% by weight) in the fuming sulfuric acid used in the reaction, and when $H_3BO_3$ is used, it is calculated from the actual content on the basis that part of the $SO_3$ reacts with the $H_3BO_3$ as follows:

$$2H_3BO_3 + 3SO_3 \rightarrow 3H_2SO_4 + B_2O_3$$

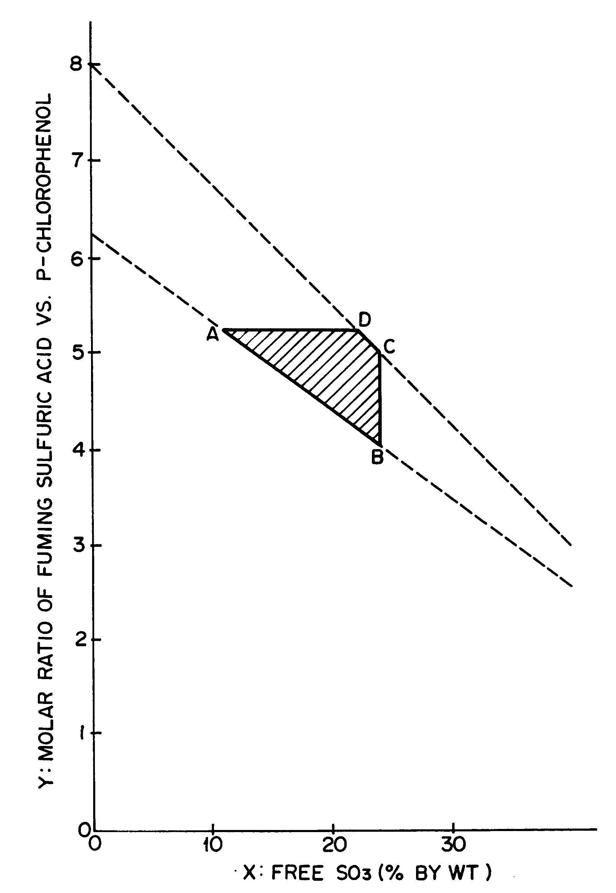

The accompanying drawing shows the relation between molar ratio of the fuming sulfuric acid against p-chlorophenol (Y) and content of the free $SO_3$ (X). The oblique lined region surrounded by ABCD in the drawing corresponds to the range where X and Y satisfy the above-mentioned formulas (1), (2) and (3)

EP 0 397 137 B1

simultaneously.

When X and Y do not satisfy the formulas (1), (2) and (3) simultaneously, yield and/or purity of the products are lowered. For example, when 8.00 - 0.125X < Y, the yield is lowered drastically. Even when, for instance, X and Y satisfy the formula (1) above but at least one of the formulas (2) and (3) are not satisfied, not only the yield of product but also purity thereof are lowered drastically. Furthermore, when too large an amount of the fuming sulfuric acid is used in the reaction and Y is larger than 5.25, the object of the present invention, particularly, using a smaller amount of the sulfuric acid, is not achieved.

The amount of the phthalic anhydride used in the reaction is not less than 1.1 mole per mole of p-chlorophenol, preferably, 1.1 - 1.5 mole, more preferably 1.1 - 1.25 mole.

The amount of boric anhydride used in the reaction is 0.6 - 1.0 mole per mole of p-chlorophenol, preferably 0.7 - 0.9 mole. In place of the boric anhydride, boric acid may be used. The amount of the boric acid used in the reaction is 1.2 - 2.0 moles per mole of p-chlorophenol, preferably 1.4 - 1.8 moles. Both boric anhydride and boric acid may be used together.

The reaction temperature of the present invention is 190 - 220°C, preferably 195 - 215°C, more preferably 200 - 210°C. Reaction time varies depending on the reaction temperature. If the reaction temperature is high, the reaction time is short. On the other hand, if the reaction temperature is low, the reaction time is long. However, if the reaction time is too long, purity of the product is decreased. Accordingly, generally speaking, the reaction time should be about 6 - 15 hours, preferably 7 - 12 hours.

Then, hydrolysis of the reaction product is effected, since the reaction product contains quinizarin boric acid ester. The hydrolysis is conducted, for example, in such a manner that the reaction product is diluted with water until the concentration of sulfuric acid is controlled to 15 - 45% by weight, and then stirred for 3 - 20 hours at 90 - 130°C under pressure or not. After the hydrolysis is over, the product is filtrated while the solution is still hot. The cake obtained is washed with warm water or weak alkaline agents such as dilute aqueous sodium hydroxide, aqueous sodium carbonate, aqueous sodium hydrogencarbonate or aqueous ammonia, and dried to obtain quinizarin with high purity and in high yield.

According to the process of the present invention, acid wasted is made small without damaging yield or purity of quinizarin Accordingly, the present invention is of great value from an industrial view.

The present invention is explained by the following nonlimiting Examples. In the Examples, part and % mean part by weight and % by weight, respectively, unless otherwise specified.

Example 1

Into a reactor which has the same volume as that of 300 parts of water (hereinafter referred to as " reactor of 300 volume parts") was charged fuming sulfuric acid (194.4 parts) containing 20% of free $SO_3$ - (hereinafter referred to as 20% fuming sulfuric acid) and the temperature was raised under stirring. When the temperature reached about 50°C, 130 - 140°C and 140 - 150°C, boric anhydride (26.6 parts), phthalic anhydride (80.0 parts) and p-chlorophenol (57.9 parts) were added, respectively. The temperature was further raised up to 205°C, and the reactor was kept at 205 ± 2°C for 11 hours. Then, the reaction mixture was cooled to 160°C and charged into water (650 parts) under stirring. The reaction mixture obtained was subjected to about 106°C for 6 hours under refluxing, and was filtered while it was hot to obtain a cake. The cake was washed with water (70°C), diluted aqueous sodium carbonate solution and water(70°C), in the order, and dried to obtain quinizarin (94.7 parts; purity: 91.5%) Yield: 80.1% based on p-chlorophenol. The results are shown in Table 1.

Examples 2 - 9 and Comparative Examples 1 - 5

Example 1 was repeated except that the amount of the fuming sulfuric acid and the content of free $SO_3$ were changed as mentioned in Table 1. The results are shown in Table 1.

Table 1

| No. | Examples | | | | | | | | | Comparative Examples | | | | |
|---|---|---|---|---|---|---|---|---|---|---|---|---|---|---|
| | 1 | 2 | 3 | 4 | 5 | 6 | 7 | 8 | 9 | 1 | 2 | 3 | 4 | 5 |
| Content of free $SO_3$ (%) | 20.0 | 20.0 | 20.0 | 20.0 | 14.0 | 14.0 | 24.0 | 24.0 | 24.0 | 20.0 | 14.0 | 24.0 | 30.0 | 33.3 |
| Amount of fuming sulfuric acid (parts) | 194.4 | 186.0 | 211.3 | 221.8 | 214.0 | 224.7 | 176.4 | 192.8 | 209.6 | 178.1 | 204.2 | 220.0 | 190.4 | 149.5 |
| Mole ratio of fuming sulfuric acid to p-chloro-phenol | 4.60 | 4.40 | 5.00 | 5.25 | 5.00 | 5.25 | 4.21 | 4.60 | 5.00 | 4.21 | 4.77 | 5.25 | 4.60 | 3.64 |
| Amount of quinizarin obtained (parts) | 94.7 | 95.3 | 94.1 | 93.2 | 95.2 | 94.5 | 95.4 | 95.1 | 93.4 | 95.3 | 94.3 | 87.3 | 92.1 | 87.7 |
| Purity of quinizarin obtained (%) | 91.5 | 90.0 | 91.7 | 91.7 | 90.0 | 91.0 | 90.5 | 90.8 | 91.5 | 84.0 | 85.9 | 91.7 | 87.4 | 87.0 |
| Yield of quinizarin based on p-chloro-phenol (%) | 80.1 | 79.3 | 79.8 | 79.0 | 79.2 | 79.5 | 79.8 | 79.8 | 79.0 | 74.0 | 74.9 | 74.0 | 74.4 | 70.5 |

Example 10

Into a reactor of 300 volume parts was charged 20% fuming sulfuric acid (194.4 parts) and the temperature was raised under stirring. When the temperature reached about 50°C, 130 - 140°C and 140 -

4

150°C, boric anhydride (26.6 parts), phthalic anhydride (83.3 parts) and p-chlorophenol (57.9 parts) were added, respectively. The temperature was raised up to 210°C, and the reactor was kept at 210 ± 2°C for 9 hours. Then, the reaction mixture was charged into water (385 parts) under stirring. The reaction mixture was subjected to about 114°C for 12 hours under refluxing, and was filtered while it was hot to obtain a cake. The cake was washed with water (70°C) until the filtrate water was neutral, and dried to obtain quinizarin (94.8 parts; purity: 91.2%). Yield: 79.9% based on p-chlorophenol.

Example 11

Into a reactor of 300 volume parts was charged 24.0% fuming sulfuric acid (176.5 parts), and the temperature was raised under stirring. When the temperature reached about 50°C, 130 - 140°C and 140 - 150°C, boric anhydride (26.6 parts), phthalic anhydride (83.3 parts) and p-chlorophenol (57.9 parts) were added, respectively. The reactor was heated up to 205°C, and was kept at 205 ± 2°C for 11 hours. Then, the reaction mixture was charged into water (598 parts) under stirring. The reaction mixture was subjected to about 106°C for 6 hours under refluxing, and was filtered while it was hot to obtain a cake. The cake was washed with water (70°C), diluted aqueous ammonia solution and water (70°C) in this order, and was dried to obtain quinizarin (95.4 parts; purity: 90.5%). Yield: 79.8% based on p-chlorophenol.

Comparative Exampel 6

Comparative Example 1 was repeated except that phthalic anhydride (73.5 parts in place of 80.0 parts), the reaction temperature (200°C in place of 205°C) and the reaction time (16 hours in place of 11 hours) were used, to obtain quinizarin (95.0 parts; purity: 83.5%). Yield: 73.3% based on p-chlorophenol.

Comparative Example 7

Comparative Example 2 was repeated except that 65% fuming sulfuric acid (204.2 parts, in place of 20% fuming sulfuric acid), boric acid (46.9 parts in place of boric anhydride (26.6 parts)), the reaction temperature (200°C in place of 205°C) and the reaction time (16 hours in place of 11 hours) were used, to obtain quinizarin (93.2 parts; purity: 86.2%). Yield: 74.3% based on p-chlorophenol.

Comparative Example 8

Comparative Example 5 was repeated except that phthalic anhydride (89.1 parts in place of 80.0 parts), boric anhydride (27.8 parts in place of 26.6 parts) and the reaction time (14 hours in place of 11 hours) were used to obtain quinizarin (88.0 parts; purity: 87.3%). Yield: 71.0% based on p-chlorophenol.

**Claims**

1. A process for preparing quinizarin which comprises allowing p-chlorophenol to react with phthalic anhydride in fuming sulfuric acid given below in the presence of boric anhydride or boric acid and hydrolyzing the resultant product, said fuming sulfuric acid satisfying the following formulas (1), (2) and (3), simultaneously,

$$6.20 - 0.090X \leq Y \leq 8.00 - 0.125X \qquad (1)$$

$$X \leq 24.0 \qquad (2)$$

$$Y \leq 5.25 \qquad (3)$$

wherein X is the content of free $SO_3$ (% by weight) in the fuming sulfuric acid, and Y is the molar ratio of the fuming sulfuric acid to the p-chlorophenol calculated according to the following equation:

$$Y = \frac{Z \left\{ \dfrac{X}{80.06} + \dfrac{100 - X}{98.08} \right\}}{\dfrac{100 \times W}{128.56}}$$

wherein

Z    is the weight of fuming sulfuric acid used in the reaction,

W    is the weight of p-chlorophenol used in the reaction,

and

X    is as defined above.

2. The process according to claim 1 wherein the amount of the phthalic anhydride is not less than 1.1 mole per mole of the p-chlorophenol.

3. The process according to claim 1 wherein the amount of the boric anhydride is 0.6 - 1.0 mole per mole of the p-chlorophenol.

4. The process according to claim 1 wherein the amount of the boric acid is 1.2 - 2.0 mole per mole of the p-chlorophenol.

5. The process according to claim 1 wherein the reaction between the p-chlorophenol and the phthalic anhydride is conducted at 190 - 220 °C.

6. The process according to claim 1 wherein the reaction time between the p-chlorophenol and the phthalic anhydride is 6 - 15 hours.

**Patentansprüche**

1. Verfahren zur Herstellung von Chinizarin, umfassend das Umsetzen von p-Chlorphenol mit Phthalsäureanhydrid in nachstehend beschriebener rauchender Schwefelsäure in Gegenwart von Borsäureanhydrid oder Borsäure und das Hydrolysieren des entstehenden Produkts, wobei die rauchende Schwefelsäure die folgenden Formeln (1), (2) und (3) gleichzeitig erfüllt

6,20 - 0,090X ≦ Y ≦ 8,00 - 0,125    (1)

X ≦ 24,0    (2)

Y ≦ 5,25    (3)

in denen X der Gehalt an freiem $SO_3$ (in Gew.-%) der rauchenden Schwefelsäure und Y das Molverhältnis der rauchenden Schwefelsäure zum p-Chlorphenol, berechnet nach der folgenden Gleichung, ist:

$$Y = \frac{Z\left\{\dfrac{X}{80,06} + \dfrac{100-X}{98,08}\right\}}{\dfrac{100 \times W}{128,56}}$$

in der

Z das Gewicht der in der Umsetzung verwendeten rauchenden Schwefelsäure ist,

W das Gewicht des in der Umsetzung verwendeten p-Chlorphenols ist, und

X wie vorstehend definiert ist.

2. Verfahren nach Anspruch 1, wobei die Menge des Phthalsäureanhydrids nicht weniger als 1,1 Mol pro Mol p-Chlorphenol beträgt.

3. Verfahren nach Anspruch 1, wobei die Menge des Borsäureanhydrids 0,6 - 1,0 Mol pro Mol p-Chlorphenol beträgt.

4. Verfahren nach Anspruch 1, wobei die Menge der Borsäure 1,2 - 2,0 Mol pro Mol p-Chlorphenol beträgt.

5. Verfahren nach Anspruch 1, wobei die Umsetzung zwischen p-Clorphenol und Phthalsäureanhydrid bei 190 - 220°C durchgeführt wird.

6. Verfahren nach Anspruch 1, wobei die Reaktionszeit zwischen p-Chlorphenol und Phthalsäureanhydrid 6 - 15 Stunden beträgt.

**Revendications**

1. Procédé pour produire quinizarine consistant à faire réagir p-chlorphénol avec un anhydride phtalique dans l'acide sulfurique fumant décrit ci-dessous en presence de l'anhydride borique ou l'acide borique et hydrolyser le produit résultant, ledit acide sulfurique fumant satisfaisant aux formules suivantes (1), (2) et (3) simultanément

$6.20 - 0.090X \leqq Y \leqq 8.00 - 0.125X$  (1)

$X \leqq 24.0$  (2)

$Y \leqq 5.25$  (3)

dans lesquelles X est le contenu de $SO_3$ libre (% en poids) dans l'acide sulfurique fumant et Y est la proportion molaire de l'acide sulfurique fumant au p-chlorphénol, calculée par l'équation suivante

$$Y = \frac{Z\left\{\dfrac{X}{80.06} + \dfrac{100-X}{98.08}\right\}}{\dfrac{100 \times W}{128.56}}$$

7

dans laquelle
Z est le poids de l'acide sulfurique fumant utilisé dans la réaction,
W est le poids du p-chlorphénol utilisé dans la réaction, et
X est tel que défini ci-dessus.

2. Procédé selon la revendication 1 dans lequel la quantité d'anhydride phtalique n'est pas inférieure à 1.1 mole par mole de p-chlorphénol.

3. Procédé selon la revendication 1 dans lequel la quantité d'anhydride borique est 0.6 - 1.0 mole par mole de p-chlorphénol.

4. Procédé selon la revendication 1 dans lequel la quantité d'acide borique est 1.2 - 2.0 mole par mole de p-chlorphénol.

5. Procédé selon la revendication 1 dans lequel la réaction du p-chlorphénol avec l'anhydride phtalique est effectuée à 190 - 220°C.

6. Procédé selon la revendication 1 dans lequel le temps de réaction du p-chlorphénol avec l'anhydride phtalique est 6 - 15 heures.